Europäisches Patentamt

European Patent Office

Office européen des brevets

(19)

(11) Numéro de publication: **0 145 548
B1**

(12)

# FASCICULE DE BREVET EUROPEEN

(45) Date de publication du fascicule du brevet:
**30.03.88**

(21) Numéro de dépôt: **84402256.6**

(22) Date de dépôt: **09.11.84**

(51) Int. Cl.⁴: **A 61 B 5/03**

(54) **Capteur de la pression intracranienne.**

(30) Priorité: **22.11.83  FR 8318548**

(43) Date de publication de la demande:
**19.06.85 Bulletin 85/25**

(45) Mention de la délivrance du brevet:
**30.03.88 Bulletin 88/13**

(84) Etats contractants désignés:
**BE CH DE GB IT LI NL SE**

(56) Documents cités:
**WO - A - 83/03190
FR - A - 2 274 261
US - A - 3 828 774**

(73) Titulaire: **Lacombe, Jacques, 17, Rue Mathis,
F-75019 Paris (FR)**
Titulaire: **Sainte-Rose, Christian, 14, Rue Corbon,
F-75015 Paris (FR)**

(72) Inventeur: **Lacombe, Jacques, 17, Rue Mathis,
F-75019 Paris (FR)**
Inventeur: **Sainte-Rose, Christian, 14, Rue Corbon,
F-75015 Paris (FR)**

(74) Mandataire: **Tony-Durand, Serge, Cabinet
Tony-Durand 77, rue Boissière, F-75116 Paris (FR)**

## Description

La présente invention concerne les capteurs utilisés pour mesurer la pression intracrânienne d'un individu.

Cette mesure présente un grand intérêt, car elle permet de déceler des pathologies du système nerveux central. En effet, dans de tels cas la circulation du liquide céphalo-rachidien est perturbée avec pour conséquence une augmentation de la pression intracrânienne. Ainsi la mesure de celle-ci permet notamment d'apprécier le retentissement d'une pathologie déterminée, de poser une indication chirurgicale ou de suivre une évolution pré ou post opératoire.

L'une des méthodes utilisées pour mesurer la pression intracrânienne consiste à introduire un cathéter dans les cavités intracérébrales en lui faisant traverser la dure-mère. Toutefois cette méthode présente des risques graves d'infections.

C'est la raison pour laquelle la méthode la plus courante consiste à pratiquer simplement un trou dans l'enveloppe osseuse du crâne de façon à accéder à l'espace situé autour de la dure-mère, puis à remplir cet espace de sérum physiologique et à mesurer la pression qui y règne en le reliant à un capteur de pression au moyen d'un tube de liaison. Cette méthode met à profit le fait que la dure-mère joue le rôle d'une membrane élastique transmettant, vers l'extérieur, la pression régnant à l'intérieur du cerveau. Cependant cette méthode impose d'obturer le trou pratiqué dans l'os du crâne afin de réaliser une cavité close et ce, en plaçant dans ce trou un bouchon portant le tube de liaison ainsi qu'un autre tube destiné à purger ensuite la cavité correspondante.

Il existe actuellement deux types de bouchons de communication utilisés à cet effet, à savoir les bouchons à vis et les bouchons conçus pour être enfoncés à force. Or ces deux types de bouchons présentent de sérieux inconvénients.

Ainsi les bouchons à vis ne peuvent être employés que si l'os du crâne est suffisamment épais, ce qui exclut leur utilisation de façon fiable pour des enfants âgés de moins de 6 ans. De plus ces bouchons fuient assez souvent et nécessitent un rinçage fréquent.

Quant aux bouchons destinés à être enfoncés à force, ils peuvent s'employer pour toutes épaisseurs de l'enveloppe crânienne. Mais sous peine de fuites, ils nécessitent l'exécution d'un trou parfaitement cylindrique, ce qui est difficile à réaliser. Par ailleurs dans les os très minces, leur enfoncement à force peut entraîner des fractures de refend.

De toutes façons, ces deux types de bouchons ont l'inconvénient de présenter des risques fréquents de fuites. Or de telles fuites sont de nature à perturber et à fausser les mesures effectuées.

C'est pourquoi la présente invention a pour but de réaliser un capteur du type en cause dans lequel le bouchon de communication, destiné à être placé dans l'os du crâne, est conçu de façon à éviter les inconvénients exposés ci-dessus.

A cet effet, le bouchon de communication de ce capteur est constitué par un corps cylindrique en matière rigide, ou semi-rigide, pourvu d'une collerette supérieure de limitation d'enfoncement et portant, à son extrémité opposée, un joint d'étanchéité en matière élastique apte à assurer l'étanchéité voulue contre la paroi du trou correspondant par flexion élastique et/ou par déformation dans son épaisseur. En outre, le joint prévu sur le bouchon de communication du présent capteur comporte une lèvre élastiquement déformable en forme de tronc de cône dont la base est tournée du côté de la collerette supérieure.

Ainsi ce bouchon de communication est en mesure d'assurer une parfaite étanchéité, même si le trou pratiqué dans l'enveloppe crânienne n'est pas parfaitement cylindrique. Par ailleurs cette étanchéité se trouve assurée dans des enveloppes crâniennes présentant des épaisseurs d'importance très variable. Ceci permet de l'employer à la fois sur des adultes et sur des enfants.

Dans ces conditions, lors de l'enfoncement de ce bouchon, la lèvre de son joint est amenée à se déformer plus ou moins en fonction de la forme exacte du trou pratiqué dans l'enveloppe du crâne et des irrégularités de la paroi interne de ce trou. Quel que soit le cas, l'élasticité de cette lèvre assure donc l'étanchéité voulue.

Cependant d'autres particularités et avantages du capteur selon l'invention apparaîtront au cours de la description suivante de quelques exemples de réalisation de celui-ci. Cette description est donnée en référence au dessin annexé à simple titre indicatif, et sur lequel:

La figure 1 est une vue en coupe axiale d'une première forme de réalisation du bouchon de communication du capteur selon l'invention;

La figure 2 est une vue similaire, mais partielle, qui illustre le mode de fonctionnement du joint prévu sur ce bouchon de communication;

La figure 3 est une vue partielle en coupe-élévation d'une autre forme de réalisation du bouchon de communication du présent capteur.

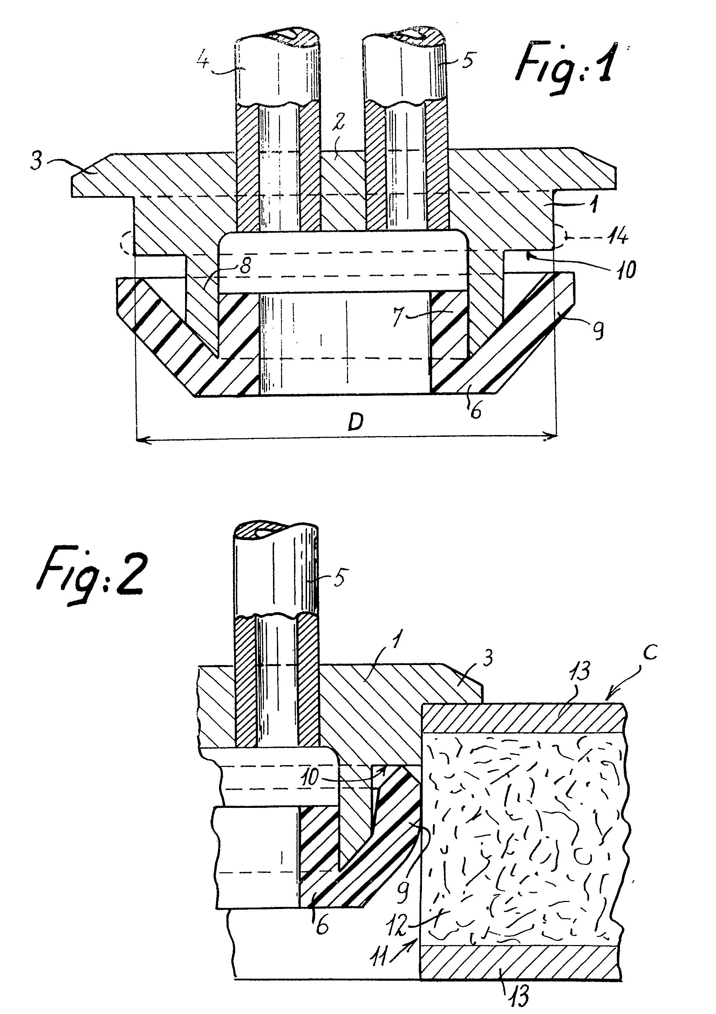

Dans l'exemple représenté aux figures 1 et 2 le bouchon de communication du capteur selon l'invention est constitué par un corps 1 de forme générale cylindrique fermé par un couvercle supérieur 2 et qui présente une collerette 3 à son extrémité supérieure. Ce corps rigide, ou semi-rigide, est avantageusement fabriqué par moulage en résine synthétique appropriée, par exemple du polyuréthane rigide.

Le couvercle 2 du corps de ce bouchon est traversé par les extrémités de deux tubes 4 et 5 débouchant à l'intérieur de la partie cylindrique du présent bouchon. L'un de ces tubes est destiné à servir d'élément de liaison avec un capteur de pression branché à son extrémité opposée. Quant à l'autre tube, il est prévu pour purger la cavité intérieure du crâne.

Sur le bord de cette jupe cylindrique, le présent bouchon porte un joint d'étanchéité 6 de forme annulaire présentant en quelque sorte une section en forme de V. La branche interne 7 de ce joint est cylindrique et son diamètre externe correspond au diamètre interne de la jupe 8 du présent bouchon. Quant à la branche externe 9 du joint 6, elle présente la forme d'un tronc de cône dont la base est tournée du côté du couvercle 2 du présent bouchon. Ce joint est fixé en place sur la jupe 8 du corps de ce bouchon par collage à la fois contre la face interne de cette jupe et contre le bord extrême de celle-ci, ce bord étant biseauté selon le même angle d'inclinaison que la branche externe 9 du joint. Le diamètre D du corps cylindrique 1 du présent bouchon détermine le diamètre du trou qui devra être pratiqué dans l'enveloppe osseuse d'un crâne pour la mise en place de celui-ci. Ce diamètre peut être de 10 mm par exemple. Quant au diamètre de la collerette 3, il peut être de 13 mm, cette collerette ayant pour but de servir de butée d'arrêt pour l'enfoncement du présent bouchon.

Il convient de noter que la jupe cylindrique 8 de celui-ci présente un dégagement 10 destiné à servir de logement, tout au moins partiel, à la lèvre externe 9 du joint 6 lors de l'enfoncement du présent bouchon dans le trou pratiqué dans un crâne. Les diamètres interne et externe de la jupe 8 peuvent être respectivement de 6 mm et de 7,5 mm pour un corps de bouchon ayant un diamètre de 10 mm.

Le joint d'étanchéité 6 est réalisé en matière élastique. Ainsi il peut être avantageusement fabriqué en polyuréthane souple.

La figure 2 illustre les conditions d'utilisation du présent bouchon de communication. Celui-ci est engagé à l'intérieur d'un trou cylindrique 11 pratiquée au préalable dans l'enveloppe osseuse C du crâne d'un patient, le diamètre de ce trou étant sensiblement égal au diamètre externe du corps 1 de ce bouchon. Lors de l'enfoncement de celui-ci la branche externe 9 du joint 6 se trouve rabattue contre la jupe cylindrique 8 du présent bouchon et ce à l'intérieur du dégagement externe 10 de cette jupe. A ce sujet, il convient de noter que le bouchon de communication selon l'invention est conçu de façon à mettre à profit la structure particulière de l'os du crâne, et plus précisément le fait que celui-ci est constitué d'une partie centrale épaisse 12 très alvéolée et friable (le diploë), disposée entre deux couches minces 13, plus compactes et plus dures (les tables interne et externe). En effet, lorsque ce bouchon est en place son corps cylindrique 1 se trouve engagé à l'intérieur de la table externe 13 et éventuellement en partie à l'intérieur du diploë 12. Par contre le joint 6 est engagé uniquement à l'intérieur du diploë. Dans ces conditions sa lèvre externe 9 vient légèrement s'imprimer dans la matière friable de celui-ci, ce qui assure une étanchéité absolument parfaite.

La dissociation du présent bouchon en deux parties de caractéristiques mécaniques différentes – à savoir: le corps 1 en matière rigide et le joint inférieur 6 en matière élastique – a non seulement pour avantage de permettre l'obtention d'une parfaite étanchéité, mais également d'éviter des perturbations dans la transmission des pressions. En effet comme le corps 1 du présent bouchon est en matière rigide il ne risque pas de déformer les variations rapides de pression pouvant se produire à l'intérieur du crâne, comme cela serait le cas pour un bouchon entièrement réalisé en matière élastique. Bien entendu les tubes 4 et 5 sont eux-mêmes fabriqués en une matière présentant une compliance appropriée pour la transmission des pressions dans les meilleurs conditions possibles.

Du fait de ses caractéristiques, le présent bouchon de communication ne présente pas les inconvénients des bouchons actuels conçus pour un enfoncement à force. Par ailleurs, il ne comporte pas les inconvénients inhérents aux bouchons actuels à vis. Cependant le principal avantage du présent bouchon est d'assurer une parfaite étanchéité sans qu'il soit nécessaire que son logement soit constitué par un trou parfaitement cylindrique. Ceci est très important car il est extrêmement difficile, sinon impossible, de réaliser un trou parfaitement cylindrique, compte tenu des difficultés pratiques d'exécution de celui-ci: perçage à main-levée, absence d'appui possible sur le crâne, caractère friable du diploë.

Eventuellement le présent bouchon de communication peut comporter, à la base de sa partie supérieure, un bourrelet annulaire 14 destiné à constituer un organe de retenue. En effet, en raison de sa position, ce bourrelet vient s'imprimer fortement dans le diploë lors de la mise en place du présent bouchon. Ceci évite donc tout risque d'arrachement intempestif. Ce bourrelet annulaire peut être réalisé d'une seule pièce avec le corps 1 du présent bouchon lors du moulage de celui-ci. Cependant il peut être également réalisé séparément et rapporté ensuite sur le corps de ce bouchon.

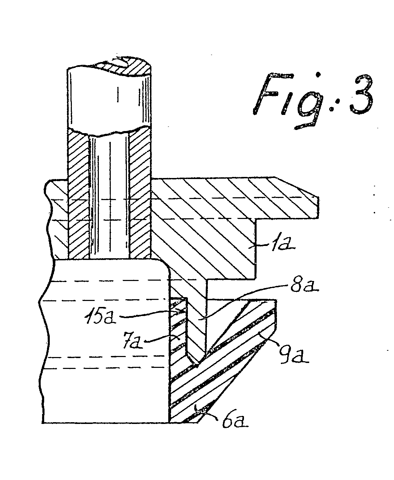

La figure 3 représente une variante de réalisation dans laquelle la branche interne 7a du joint élastique correspondant 6a est encastrée dans un logement 15a prévu sur la face interne de la jupe cylindrique 8a du bouchon correspondant 1a. L'agencement ainsi prévu a pour avantage que la branche interne 7a de ce joint ne fait pas saillie sur la paroi interne de la jupe 8a. Mais par ailleurs la structure du corps du bouchon et celle du joint est la même que précédemment. En effet la branche externe 9a de ce joint constitue une lèvre en forme de tronc de cône apte à assurer l'étanchéité voulue.

**Revendications**

1. Capteur de la pression intracrânienne, comportant un bouchon de communication destiné à être mis en place dans un trou pratiqué dans l'enveloppe du crâne et qui porte des tubes de communication avec l'intérieur du crâne, ce bouchon étant constitué par un corps cylindrique (1) en matière rigide, ou semi-rigide, pourvu d'une collerette supérieure (3) de limitation d'enfoncement, caractérisé en ce que ce bouchon porte, à son extrémité opposée à cette collerette, un joint d'étanchéité (6) en matière élastique comprenant une lèvre (9 ou 9a) élastiquement déformable en forme de tronc de cône dont la base est tournée du côté de la collerette supérieure (3), ce joint étant apte à assurer l'étanchéité voulue contre la paroi du trou correspondant du fait que ce joint vient s'imprimer élastiquement dans la partie friable (diploë) de cette paroi.

2. Capteur selon la revendication 1, caractérisé en ce que la paroi de la jupe cylindrique (8) de son bouchon de communication (1) comporte, sur la face externe, un dégagement (10) apte à servir de logement, au moins partiel, à cette lèvre tronconique, lors de sa déformation résultant de la mise en place de ce bouchon dans le trou correspondant.

3. Capteur selon l'une des revendications précédentes, caractérisé en ce que le corps de son bouchon de communication, porte à sa partie supérieure, un bourrelet annulaire (14) destiné à s'imprimer dans la paroi du trou à l'intérieur duquel est engagé ce bouchon, afin de constituer un organe de retenue de celui-ci.

## Patentansprüche

1. Intrakranialdrucksensor mit einem Kommunikations-Verschlussstück zum Einsetzen in ein in der Hirnschale angebrachtes Loch und mit Röhren zur Verbindung mit dem Schädelinneren, wobei das Verschlussstück einen zylindrischen Körper (1) aus steifem oder halbsteifem Werkstoff aufweist und mit einem oberen Begrenzungsrand (3) zur Begrenzung der Eindringtiefe versehen ist, dadurch gekennzeichnet, dass das Verschlussstück an seiner, besagtem Begrenzungsrand gegenüberliegenden Extremität, eine Dichtung (6) aus elastischem Werkstoff mit einer Lippe (9 oder 9a) aufweist, die elastisch deformierbar in Form eines Kegelstumpfes ausgebildet ist, dessen Basis auf der dem oberen Begrenzungsrand (3) zugewandten Seite liegt, wobei diese Dichtung geeignet ist, die gewünschte Dichtigkeit gegenüber der Lochwand sicherzustellen, und zwar dadurch, dass sich diese Dichtung elastisch gegen den bröckeligen Teil (Diploe) andrückt.

2. Sensor nach Anspruch 1, dadurch gekennzeichnet, dass die Wand des zylindrischen rockförmigen Teils (8) des Kommunikations-Verschlussstückes (1) an seiner Aussenseite eine Ausnehmung bzw. einen freien Raum (10) aufweist, welcher geeignet ist, zur wenigstens teilweisen Aufnahme besagter kegelstumpfförmigen Lippe zu dienen, wenn deren Deformation infolge des Einsetzens des Verschlussstückes in das entsprechende Loch erfolgt.

3. Sensor nach einem der vorhergehenden Ansprüche, dadurch gekennzeichnet, dass der Körper des Kommunikations-Verschlussstückes an seinem oberen Teil einen ringförmigen Wulst (14) aufweist, der dazu bestimmt ist, sich in die Wand des Loches einzudrücken, in dessen Inneres das Verschlussstück eingesetzt ist, um ein Rückhalteorgan für dieses zu bilden.

## Claims

1. Intracranial pressure sensor comprising a communication plug which is intended to be inserted in a hole formed in the skull-case and which carries tubes for providing a communication with the interior of the skull, this plug being constituted by a cylindrical body (1) of rigid or semi-rigid material provided with a top annular flange (3) for limiting the depth of penetration, characterized in that this seal carries at the end opposite to this annular flange a seal (6) of elastic material having an elastically deformable lip (9 or 9a) of frusto-conical shape, the base of which is directed towards the top annular flange (3) this seal being capable of ensuring the requisite leaktightness against the wall of the corresponding hole by virtue of the fact that this seal is elastically impressed into the friable portion (diploe) of this wall.

2. Sensor in accordance with claim 1, characterized in that the wall of the cylindrical skirt (8) of its communication plug (1) is provided on the external face with a recess (10) which is capable of serving at least as a partial housing for this frustoconical lip at the time of its deformation which results from positioning of this plug within the corresponding hole.

3. Sensor in accordance with one of the preceding claims, characterized in that the body of its communication plug carries at the top portion thereof an annular bead (14) which is intended to be impressed into the wall of the hole within which this plug is engaged in order to constitute an element for retaining this latter.

1/2

*Fig:1*

*Fig:2*

2/2

Fig. 3